# EUROPÄISCHE PATENTSCHRIFT

(11) **EP 2 786 771 B1**
(45) Veröffentlichungstag und Bekanntmachung des Hinweises auf die Patenterteilung: **06.12.2017**
(21) Anmeldenummer: 14157250.3
(22) Anmeldetag: 28.02.2014
(51) Int. Cl.: A61L 27/04, A61L 27/10, A61L 27/30, A61L 27/50

(54) **Verfahren zur Herstellung eines funktionalisierten Implantats sowie funktionalisiertes Implantat**
Method for the preparation of a functionalised implant and functionalised implant
Procédé de fabrication d'un implant fonctionnalisé et implant fonctionnalisé

(30) Priorität: 11.03.2013 DE 102013102370
(43) Veröffentlichungstag der Anmeldung: 08.10.2014
(60) Teilanmeldung: 16165110.4
(73) Patentinhaber: DOT GmbH, 18059 Rostock (DE)
(72) Erfinder: Neumann, Hans-Georg, 18146 Rostock (DE); Lembke, Ulrich, 18147 Rostock (DE)
(74) Vertreter: Prinz & Partner mbB

(56) Entgegenhaltungen:
- WO-A1-2012/011878
- WO-A2-2009/036845
- US-A1- 2003 153 981

## Beschreibung

Die Erfindung betrifft ein Verfahren zur Herstellung eines funktionalisierten Implantats, insbesondere eines Dentalimplantats, sowie ein funktionalisiertes Implantat.

In den vergangenen Jahrzehnten sind die Anforderungen, die Ärzte und Patienten an die Funktionalität von Implantaten stellen, beständig gestiegen. Dabei spielt die ansteigende Lebenserwartung der Menschen eine entscheidende Rolle, da diese den Bedarf an biokompatiblen medizinischen Implantaten erhöht. Aufgrund dessen ist eine neue Generation von Implantaten nötig, die die Fähigkeit aufweist, schneller in den Knochen einzuwachsen und eine bessere Verbindung mit dem Knochen einzugehen. Ferner sollen derartige moderne Implantate mechanisch stabil sein und sich optimal sowie in kürzester Zeit mit dem körpereigenen Gewebe verbinden, wobei keine Abstoßungsreaktion oder gar Infektion hervorgerufen werden soll.

Es ist dabei bekannt, dass eine optimierte Anpassung insbesondere der Implantatoberfläche an das komplexe biologische Umfeld besonders wichtig ist. Diese Anpassung gelingt vielfach durch eine Modifizierung der Implantatoberflächen, beispielsweise durch eine Beschichtung, die es ermöglicht, die Oberflächeneigenschaften unabhängig von den Eigenschaften des Implantatwerkstoffs zu gestalten. Gerade die Oberflächeneigenschaften der Implantate sind von besonderer Bedeutung, da es bekannt ist, dass Zell- und Bakterienadhäsionsprozesse stark von der Oberflächenbeschaffenheit des Implantats abhängig sind. Dabei spielen vor allem die chemische Zusammensetzung sowie die Rauigkeit der Oberfläche eine erhebliche Rolle.

Eine Vielzahl der derzeit hergestellten Implantate besteht aus Titan oder einer Titanlegierung, da Titan sich seit langem als biokompatibles Material bewährt hat. Titan weist eine hohe mechanische Festigkeit auf und zeichnet sich darüber hinaus durch seine hervorragende Biokompatibilität aus. Nachteilig bei diesem Werkstoff ist hingegen, dass es lange dauert, bis eine feste Verbindung zwischen dem Knochen und dem Implantat erhalten wird, also das Implantat eingewachsen ist.

Es ist ferner bekannt, dass bei Implantaten, die für orthopädische Zwecke verwendet werden, die Oberflächen modifiziert werden, sodass eine optimale Integration der orthopädischen Implantate erreicht werden kann.

Implantate der vorstehend erläuterten Art weisen eine metallische Oberfläche auf, sodass sie aus ästhetischen Gründen für den Dentalbereich nicht die optimale Lösung darstellen, da die Farbgebung sich deutlich von den natürlichen Farben des Zahnes bzw. des Zahnfleisches unterscheidet. Im Falle einer Periimplantitis kann der Implantathals infolge des Zurückweichens des Zahnfleisches freigelegt werden, sodass das metallische Implantatmaterial an dieser Stelle dunkel durchschimmert, was kosmetisch inakzeptabel ist.

Daher werden im Dentalbereich zunehmend Implantate verwendet, die aus einem Keramiksubstrat als Basiskörper aufgebaut sind. Diese keramischen Implantatwerkstoffe weisen ebenfalls eine hohe Festigkeit sowie Biokompatibilität auf. Jedoch ist die Integration des Implantats in den Knochen im Vergleich zu einem Implantat aus einem Titanwerkstoff schwieriger. Dies liegt vor allem an den nicht vorhandenen osteokonduktiven Eigenschaften, die das Anwachsen von Osteoblasten auf der Oberfläche erleichtern würden. Daher werden die Zelladhäsionsprozesse beeinträchtigt, und es kommt zu Lockerungen der eingesetzten Implantate aufgrund einer unzureichenden knöchernen Verbindung der Implantatoberfläche mit dem Knochen. Diese mangelnde Haftung kann durch das zusätzliche Einbringen von Knochenzement allenfalls unwesentlich verbessert werden. Zudem ist auch die Haftung von Knochenzementen auf Keramikmaterialien im Vergleich zu Titanwerkstoffen deutlich reduziert.

Zur Funktionalisierung eines aus einem Keramikwerkstoff hergestellten Implantats gibt es im Stand der Technik mehrere Wege, die jedoch allesamt keine zufriedenstellenden Ergebnisse liefern. Zum einen gibt es Bestrebungen, Titan oder Titanlegierungen auf keramische Werkstoffe aufzubringen, um so die Vorteile beider Materialien miteinander zu kombinieren. Dies wird auch dadurch erschwert, dass sich die beiden Werkstoffe lediglich mechanisch miteinander verbinden, wobei keine chemische Verbindung zwischen den beiden Werkstoffen stattfindet. Daher ist es nötig, die Keramikoberfläche zunächst aufzurauen, um so eine möglichst gute mechanische Verbindung zwischen dem Keramikimplantat und der Titan- bzw. der Titanlegierung-Beschichtung herzustellen.

Ferner ist aus der WO 2009/036845 A1 ein Verfahren zur Aufbringung einer Titanlegierung auf einem Keramiksubstrat bekannt. Die Titanlegierung wird dabei mittels Plasmaspritzen auf das Keramiksubstrat aufgetragen, sodass zumindest zufriedenstellende Haftzugfestigkeiten erreicht werden. Das hierfür benötigte Keramikimplantat weist jedoch eine entsprechende Rauigkeit auf, die die Haftung des aufgespritzten Titans ermöglicht. Somit ist bei diesem bekannten Verfahren die Vorbehandlung des Keramikwerkstoffs nötig, um die gewünschte Haftzugfestigkeit zu garantieren.

Aus der US 2003/153981 A1 ist ein Verfahren bekannt, bei dem eine Struktur aus einem metallischen Schaum ausgebildet wird, die mittels eines Sinter-Verfahrens mit einem Substrat verbunden wird.

In der WO 2012/011878 A1 ist ein Verfahren zur Herstellung eines Implantats mit einer mehrschichtigen Beschichtung beschrieben, bei dem eine Titanlegierung auf einem metallischen Substrat mittels eines Plasmasprühverfahrens aufgebracht wird.

Aufgabe der Erfindung ist es, ein verbessertes Verfahren für die Herstellung eines funktionalisierten Implantats bereitzustellen, welches die schnelle, kraftschlüssige Biologisierung einer Implantatoberfläche erlaubt.

Die Aufgabe wird erfindungsgemäß durch ein Verfahren zur Herstellung eines funktionalisierten Implantats gelöst, insbesondere eines Dentalimplantats, mit den Schritten des Bereitstellens eines Substrats für das Implantat und dem Aufbringen einer Beschichtung, die mindestens eines der folgenden Materialien zur Funktionalisierung enthält: Zirkon, Titan, Zirkon- oder Titan-Legierung, wobei die Zirkon- oder Titan-Schicht oder Zirkon- oder Titan-Legierung mittels eines PVD-Verfahrens direkt auf die Oberfläche des Substrats aufgetragen wird. Mithilfe des erfindungsgemäßen Verfahrens kann somit ein funktionalisiertes Implantat, insbesondere Dentalimplantat, in einfacherer Weise hergestellt werden, wobei die nötigen Haftzugfestigkeiten dennoch erreicht werden. Das Verfahren ist dahingehend vereinfacht, dass das Substrat vor dem Beschichten bzw. dem Aufbringen der Beschichtung nicht vorbehandelt (insbesondere nicht aufgeraut) werden muss. Eine Aufrauung kann jedoch durchgeführt werden, um die Haftfestigkeit zu verbessern.

Mit dem Auftragen dieser Schicht wird eine erste Biologisierung des Implantats erreicht. Das Aufbringen der Schicht mit diesem Verfahren ist von Vorteil, da bei relativ niedrigen Substrattemperaturen gearbeitet wird. Dadurch ist gewährleistet, dass die Eigenschaften der Beschichtung sowie des Keramiksubstrats bei dem Beschichtungsprozess nicht verändert werden. Das PVD-Verfahren garantiert, dass eine haftfeste Schicht erreicht wird, da die Eigenenergie der schichtbildenden Teilchen groß genug ist. Die Substrattemperatur ist bei dem PVD-Verfahren typischerweise < 250 °C, insbesondere < 200 °C. Durch die geringe Temperatur werden die Oberfläche der Schicht bzw. Legierung und die Struktur des Grundmaterials nicht verändert, sodass das PVD-Verfahren ein schonendes Verfahren darstellt, welches die mechanischen Eigenschaften des Keramikwerkstoffes nicht beeinträchtigt. Die so erreichte Schichtdicke beträgt ca. 50 bis 150 nm, insbesondere 80 bis 100 nm.

Das Substrat kann aus einem Keramikwerkstoff bestehen, der aus einer ATZ-Keramik oder aus einer Zirkonoxid dominierten Keramik mit einem geringen Zusatz Yttriumoxid hergestellt ist, oder dessen Hauptbestandteil Aluminiumoxid ist, wobei diese Keramik durch Zirkonoxidzusätze verstärkt ist (ZTA-Keramik). Alternativ kann auch ein Substrat vorgesehen sein, das aus Metall besteht.

Durch das Aufbringen der hochporösen Beschichtung wird eine Funktionalisierung des Implantats, insbesondere der Oberfläche des Implantats, erreicht, wobei die Funktionalisierung insbesondere eine Biologisierung darstellt. Derartig funktionalisierte Implantate weisen eine mikro- und makroporöse Oberfläche auf, die zudem biokompatibel zum Knochengewebe ist. Die hochporöse Oberfläche verbessert die Einlagerung von bzw. Besiedlung durch Zellen, was wiederum den Einwachsprozess des Implantats beschleunigt. Die Funktionalisierung/Biologisierung der Implantatoberfläche wird dadurch verbessert, dass die Beschichtung eine große freie Oberfläche schafft, die zu einer schnellen und vollständigen Benetzung der Implantatoberfläche mit Körperflüssigkeit, beispielsweise Blut, führt. Auf diese Weise wird die Benetzbarkeit der Oberfläche, die ein wesentlicher Faktor für die biologische Aktivität darstellt, erheblich verbessert. Die in der Beschichtung vorhandenen Calciumionen stimulieren zudem das Wachstum der Zellen, sodass das Einwachsen des Implantats verbessert wird.

Alternativ zum PVD-Verfahren ist es möglich, die Zirkon- oder Titan-Schicht oder Zirkon- oder Titan-Legierung mittels eines CVD-Verfahrens auf das Substrat aufzubringen. Die hierbei herrschenden Temperaturen sind jedoch um einiges höher als beim PVD-Verfahren, sodass es möglicherweise zu Eigenschaftsänderungen des Verbunds und insbesondere des Keramikwerkstoffes/Grundmaterials kommen kann.

Insbesondere ist vorgesehen, dass abschließend das beschichtete Substrat in eine Natriumhydroxid-Lösung eingelegt wird. Das Einlegen in die Natriumhydroxid-Lösung verbessert die osteokonduktiven Eigenschaften des Implantats, da die Zirkon- oder Titan-Schicht bzw. -Legierung zu Zirkonoxid oder Titandioxid reagiert. Durch das Einlegen des beschichteten Implantats in die Natriumhydroxid-Lösung werden die Zirkon- bzw. Titanschichten in hochporöse und hoch hydrophile Zirkonoxid- bzw. Titanoxidschichten umgewandelt. Bei der Natriumhydroxid-Lösung handelt es sich um eine 0,2 - 10 molare, insbesondere 0,5 - 5 molare, Lösung.

Ferner ist erfindungsgemäß ein funktionalisiertes Implantat vorgesehen, insbesondere Dentalimplantat, mit einer funktionalisierten, haftfesten Beschichtung, die zumindest Zirkon, eine Legierung mit diesem Material oder Zirkonoxid zur Funktionalisierung des Implantats aufweist. Ein derartiges Implantat zeichnet sich dadurch aus, dass die biologische Aktivität deutlich verbessert ist, da es sich bei der aufgetragenen Beschichtung um eine mikro- und makroporöse bzw. hochporöse Beschichtung handelt. Eine derartige Schicht ist lediglich durch einen schonenden Auftragungsprozess herzustellen.

Bei dem Substrat kann es sich um ein Oxid-Keramik-Substrat oder um ein Substrat aus Metall handeln.

Die Beschichtung mittels einer Zirkonschicht gemäß dem zuvor genannten Verfahren stellt bereits eine Funktionalisierung des Implantats dar, da die Benetzbarkeit der Oberfläche und somit die biologische Aktivität erhöht ist. Die derartige Beschichtung weist eine Dicke von 50 bis 150 nm auf.

Weitere Vorteile und Merkmale ergeben sich aus der nachfolgenden Beschreibung, in der verschiedene, beispielhafte und somit nicht einschränkende Ausführungsformen sowie deren Herstellung beschrieben werden, sowie den nachfolgenden Zeichnungen, auf die Bezug genommen wird.

Die Zeichnungen zeigen in:
- Figur 1 eine REM-Aufnahme eines ZTA-Keramik-Implantats mit einer hochporösen, hoch hydrophilen Calciumtitanat-Schicht, die mittels eines Verfahrens hergestellt worden ist,
- Figur 2 eine REM-Aufnahme eines ZTA-Keramik-Implantats mit einer hochporösen und hoch hydrophilen Titanoxidschicht, die mittels eines erfindungsgemäßen Verfahrens hergestellt worden ist, wobei vor der NaOH-Umwandlung keine Calciumphosphat-Schicht aufgebracht wurde,
- Figur 3 in einem ersten Flussdiagramm den Ablauf eines erfindungsgemäßen Verfahrens und eines Verfahrens, und

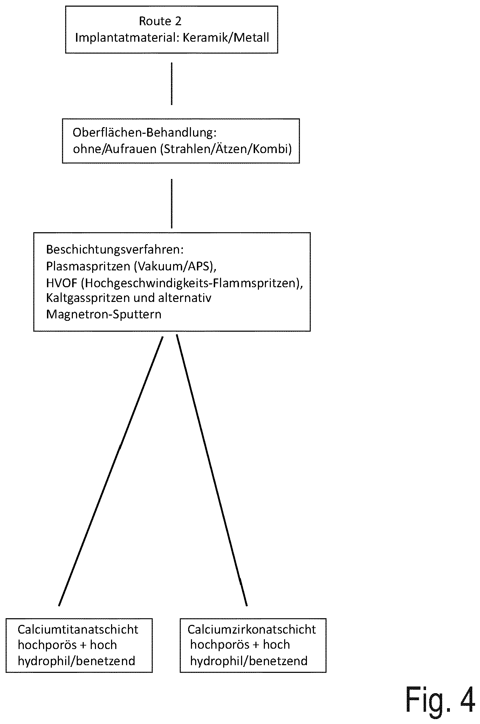

- Figur 4 in einem zweiten Flussdiagramm den Ablauf eines weiteren Verfahrens.

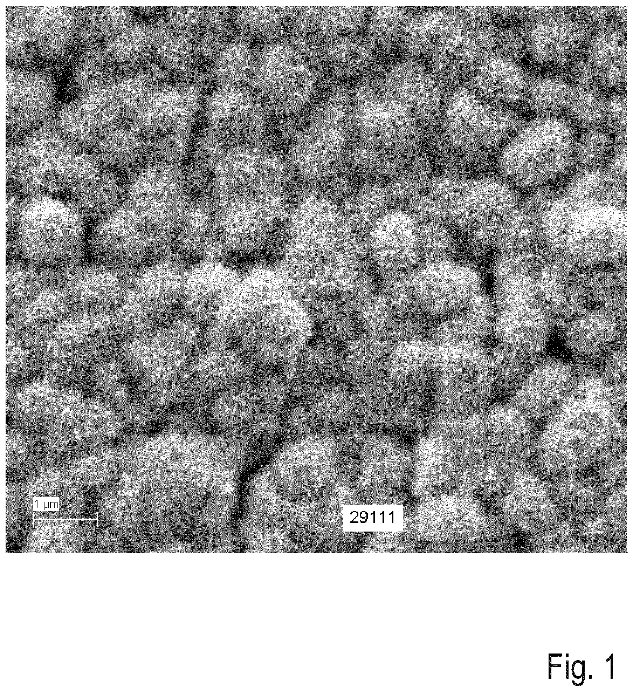

In Figur 1 ist eine REM-Aufnahme von einem funktionalisierten Dentalimplantat gezeigt, das ein aus einem Oxid-Keramik-Werkstoff hergestelltes Substrat aufweist, wobei der Oxid-Keramik-Werkstoff beispielsweise eine ZTA-Keramik (Zircona Toughened Alumina) ist. Eine derartige Keramik zeichnet sich dadurch aus, dass der Hauptbestandteil Aluminiumoxid ist, wobei Zirkonoxidzusätze zur Verstärkung vorgesehen sind. Dieses Substrat wird zur Funktionalisierung/ Biologisierung mit einer Titan-Schicht sowie einer Calciumphosphat-Schicht versehen, wobei beide Schichten nachfolgend in einem chemischen Prozess in eine hochporöse Calciumtitanatschicht umgewandelt werden.

Das Substrat wird dabei zunächst direkt, das heißt ohne Vorbehandlung, mit einer Titan-Schicht versehen. Damit sich eine haftfeste Beschichtung der Substratoberfläche ausbildet, wird die Titan-Schicht mittels eines PVD-Verfahrens auf die Oberfläche des Substrats aufgebracht.

Dies stellt den Beginn der Biologisierung dar, da bereits durch das derartige Auftragen der dünnen Schicht aus Titan die Benetzbarkeit der Oberfläche und somit die biologische Aktivität ganz erheblich verbessert wird. Bei diesem Verfahren treten Temperaturen von unter 200 °C auf, sodass keine Gefahr besteht, dass sich bei dem Auftragungsprozess die Eigenschaften des Systems Keramik/Beschichtung sowie die Oberflächeneigenschaften verändern. Trotz der geringen Substrattemperatur weisen die schichtbildenden Teilchen eine genügend hohe Eigenenergie auf, um eine haftfeste Schicht auszubilden. Die derart aufgetragene Titan-Schicht weist eine Dicke von ca. 50 bis 150 nm auf. Das derart beschichtete Implantat weist bereits jetzt eine Teilbiologisierung/-funktionalisierung auf, da die derart aufgebrachte Titan-Schicht die osteokonduktiven Eigenschaften der Oberfläche des Implantats erheblich erhöht.

Zur Verbesserung des Einwachsprozesses des Implantats wird noch eine Calciumphosphat-Schicht aufgetragen. Diese Calciumphosphat-Schicht ist aufgrund ihrer Mikro- und Makroporosität besonders gut geeignet, um das Einwachsen des Implantats in den Knochen zu beschleunigen. Des Weiteren begünstigt die Freisetzung von Calcium- und Phosphationen aus der Calciumphosphat-Schicht die Osteointegration, sodass eine schnelle und kraftschlüssige Verbindung zwischen dem Implantat und dem Biosystem hergestellt wird. Somit wird dank der Calciumphosphat-Schicht das Einwachsverhalten auch bei geringer Knochenqualität verbessert bzw. beschleunigt. Zudem wird aufgrund dessen eine höhere Toleranz gegenüber Mikrobewegungen ermöglicht. Die Calciumphosphat-Schicht wird dabei auf das Substrat mit darauf beschichteter Titan-Schicht aufgebracht, sodass sich eine dünne, bioaktive Calciumphosphat-Schicht ausbildet.

Zur Herstellung einer makroporösen bzw. hochporösen Calciumphosphat-Schicht wird das Calciumphosphat bevorzugt mittels eines elektrochemischen Prozesses auf die leitfähige Oberfläche der Titan-Schicht aufgebracht. Hierfür eignet sich insbesondere eine Elektrolyse bzw. ein elektrolytisches Bad. Als Calciumphosphat kommt insbesondere Bruschit infrage, da dieses bereits im Knochen natürlich vorkommt und in der Lage ist, den Körper kurzfristig zur eigenen Knochensynthese anzuregen, womit das knöcherne Einwachsen von Implantaten besonders in der primären Phase beschleunigt wird. Im weiteren Verlauf des Einwachsens des Implantats werden dann die leichter löslichen Bestandteile der Calciumphosphat-Schicht in das körpereigene geringer lösliche Hydroxylapatit umgewandelt.

Durch die Beschichtung des Implantats mit der Calciumphosphat-Schicht mittels eines elektrochemischen Prozesses, insbesondere durch Elektrolyse, kommt es an der Oberfläche des Implantats zur Präzipitation von Calciumphosphat aus dem lokal übersättigten Elektrolyten, sodass sich eine mikrokristalline, hochporöse und bioaktive Calciumphosphat-Schicht auf der Oberfläche ausbildet. Dank des elektrolytischen Bads ist es zudem möglich, eine komplette Bedeckung poröser Implantatoberflächen und komplizierter Geometrien zu erreichen. Ferner wird dank des elektrochemischen Prozesses die von dem Substrat vorgegebene Porosität im Gegensatz zum Beschichten mittels Plasmaspray-Verfahren nicht verringert. Aufgrund der Schichtabscheidung bei geringen Temperaturen kommt es beim Aufbringen der Calciumphosphat-Schicht mittels des elektrochemischen Verfahrens auch nicht zu Struktur- und Eigenschaftsveränderungen des Keramik-Grundmaterials. Dank der elektrochemischen Abscheidung der bioaktiven Calciumphosphat-Schichten unter annähernd physiologischen Bedingungen wird somit eine ganz neue Oberflächenqualität des Implantats erreicht.

Im nachfolgenden Prozessschritt reagiert die so auf das Implantat aufgebrachte Calciumphosphat-Schicht in einer NaOH-Lösung mit der darunterliegenden Titan-Schicht derart, dass sich die beiden Phasen zu Calciumtitanat umwandeln. Die Calciumtitanat-Schicht weist nadelförmige Kristallite auf, die vliesartig angeordnet sind. Dadurch wird eine große freie Oberfläche geschaffen, die der Implantatoberfläche eine hohe Kapillarwirkung verleiht. Dies führt zu einer schnellen und vollständigen Benetzung der Implantatoberfläche mit Körperflüssigkeit (insbesondere Blut), wodurch die biologische Aktivität nochmals erhöht wird. Diese Oberflächenbeschaffenheit ist gut in Figur 1 zu erkennen.

Das in Figur 1 dargestellte Implantat ist beispielsweise 24 Stunden lang einer 5 molaren Natriumhydroxid-Lösung bei 50 °C ausgesetzt gewesen. Anschließend wurde das Implantat gewässert sowie für 1 Stunde bei 600 °C getempert, um die gezeigte Oberfläche zu erhalten.

Aus der Calciumtitanat-Schicht eluieren die das Wachstum der Zellen stimulierenden Calciumionen, sodass ein Einwachsen des Implantats in die Umgebung beschleunigt wird. Ferner wird aufgrund der hochporösen Morphologie der Calciumtitanatschicht das Anhaften von Zellen begünstigt, was wiederum den Einwachsprozess entsprechend beschleunigt.

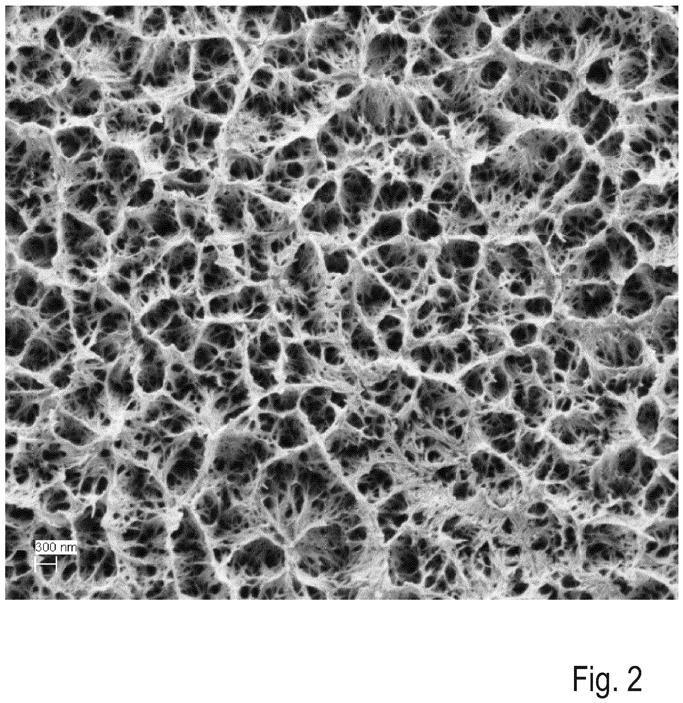

In Figur 2 ist eine REM-Aufnahme eines erfindungsgemäßen Implantats dargestellt, das ähnlich zum zuvor beschriebenen Implantat behandelt worden ist, wobei jedoch keine Calciumphosphat-Schicht aufgetragen worden ist. Bei dem in Figur 2 gezeigten Implantat handelt es sich um ein Substrat aus einer ZTA-Keramik, das analog zu dem zuvor beschriebenen Implantat mit einer Titan-Schicht mittels eines PVD-Verfahrens beschichtet worden ist. Zur Funktionalisierung ist das derart hergestellte Implantat mit einer Natriumhydroxid-Lösung bearbeitet worden, sodass auf der Oberfläche vorhandene Titan-Ionen zu Titandioxid reagieren, wodurch die osteokonduktiven Eigenschaften der Oberfläche noch einmal erheblich verbessert werden. Die Verbesserung der osteokonduktiven Eigenschaften ist auf die durch das Einlegen in die Natriumhydroxid-Lösung entstehende Hochporosität zurückzuführen.

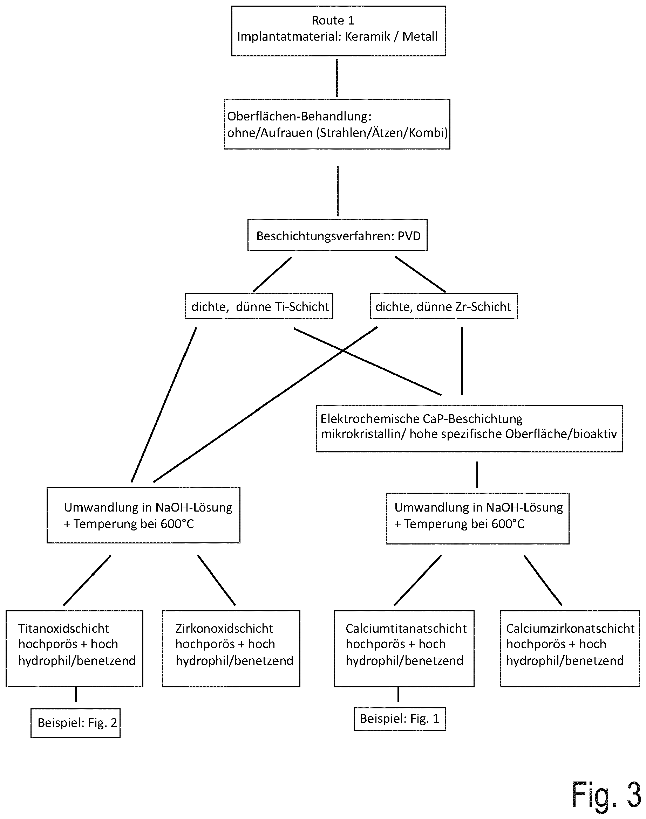

Die in Figur 1 und 2 gezeigten Implantate finden sich in der ersten Teilübersicht aus Figur 3 wieder, wobei das erfindungsgemäße Herstellungsverfahren die linke Seite der Route 1 darstellt.

Ferner kann ein Substrat für ein Implantat verwendet werden, welches aus einem Oxid-Keramik-Werkstoff besteht, der eine zirkonoxiddominierte Keramik mit einem geringen Zusatz von Yttriumoxid ist. Generell können Substrate verwendet werden, die aus einer reinen oder Mischoxid-Keramik bestehen.

Da eine Oberfläche des Substrats vorliegen kann, deren Beschaffenheit für die Besiedlung von Zellen ungünstig ist, kann die Oberfläche abrasiv bearbeitet werden. Dies bedeutet, dass eine Aufrauung der Oberfläche stattfindet, wobei dies bevorzugt durch Strahlen mit Edelkorund vollzogen wird.

Alternativ kann die raue Oberfläche auch durch Strahlen mit anderen Partikeln, wie etwa Glaskugeln oder Hydroxylapatit, sowie durch Säureätzen mit HF oder Mischsäuren oder durch eine Kombination aus Ätzen und Strahlen hergestellt werden.

Das Aufrauen durch Partikelstrahlen ist jedoch zu bevorzugen, da dies zum einen kostengünstiger und zum anderen produktionstechnisch einfacher umsetzbar ist; die rückstandslose Entfernung von Säureresten ist ein aufwändiger Prozess.

Die Aufrauungsprozesse haben allesamt gemeinsam, dass die Substratoberfläche eine vergrößerte Kontaktfläche zum Knochen aufweist, wodurch das Anhaften, insbesondere das mechanische Anhaften, von weiteren Beschichtungsbestandteilen verbessert wird.

Die Ausführungsformen haben allesamt gemeinsam, dass eine hochporöse sowie hoch hydrophile bzw. benetzende Implantatoberfläche entsteht.

Die erfindungsgemäßen Ausführungsformen haben mikroproöse, hoch hydrophile Implantatoberflächen gemeinsam, die zum einen dadurch entstehen, dass sehr dünne, aber dichte metallische Titan- oder Zirkonbasisschichten auf keramischen Implantaten abgeschieden und in NaOH zu hochporösen Titan- bzw. Zirkon-Oxidschichten umgewandelt werden.

## Patentansprüche

1. Verfahren zur Herstellung eines funktionalisierten Implantats, insbesondere Dentalimplantats, mit den Schritten:
a) Bereitstellen eines Substrats für das Implantat,
b) Aufbringen einer hochporösen, hoch hydrophilen Beschichtung, wobei zunächst mindestens eines der folgenden Materialien zur Funktionalisierung aufgebracht wird:
- Zirkon, Titan,
- Zirkon- oder Titan-Legierung, und wobei
die Zirkon- oder Titan-Schicht oder Zirkon- oder Titan-Legierung mittels eines PVD-Verfahrens direkt auf die Oberfläche des Substrats aufgetragen wird.

2. Verfahren nach einem der vorhergehenden Ansprüche, **dadurch gekennzeichnet, dass** abschließend das beschichtete Substrat in eine NaOH-Lösung eingelegt wird, wodurch die chemische Umwandlung der Schichten in Titanoxid oder Zirkonoxid erfolgt.

3. Verfahren nach einem der vorhergehenden Ansprüche, **dadurch gekennzeichnet, dass** als Substrat eine Keramik verwendet wird, insbesondere eine ATZ-Keramik, eine ZTA-Keramik oder eine Zirkonoxid dominierte Keramik mit einem Yttriumoxid-Zusatz.

4. Verfahren nach Anspruch 1 oder 2, **dadurch gekennzeichnet, dass** als Substrat ein Metall verwendet wird.

5. Funktionalisiertes Implantat, insbesondere Dentalimplantat, mit einem Substrat und einer funktionalisierten, haftfesten sowie hydrophilen Beschichtung, die zumindest Zirkon, eine Legierung mit diesem Material oder Zirkonoxid aufweist.

6. Funktionalisiertes Implantat nach Anspruch 5, **dadurch gekennzeichnet, dass** als Substrat eine Keramik verwendet wird, insbesondere eine ATZ-Keramik, eine ZTA-Keramik oder eine Zirkonoxid dominierte Keramik mit einem Yttriumoxid-Zusatz, oder ein Metall.

## Claims

1. A method of manufacturing a functionalized implant, in particular a dental implant, comprising the steps of:
a) providing a substrate for the implant;
b) applying a highly porous, highly hydrophilic coating, wherein at first at least one of the following materials is applied for functionalization:
- zirconium, titanium,
- zirconium or titanium alloy, and wherein
the zirconium or titanium layer or zirconium or titanium alloy is applied directly to the surface of the substrate by means of a PVD method.

2. The method according to any of the preceding claims, **characterized in that** the coated substrate is finally placed in a NaOH solution, whereby the layers are chemically converted to titanium oxide or zirconium oxide.

3. The method according to any of the preceding claims, **characterized in that** a ceramic material, in particular an ATZ ceramic, a ZTA ceramic, or a zirconium oxide dominated ceramic with an yttrium oxide addition, is used as the substrate.

4. The method according to claim 1 or 2, **characterized in that** a metal is used as the substrate.

5. A functionalized implant, in particular a dental implant, comprising a substrate and a functionalized, adhesive and hydrophilic coating which includes at least zirconium, an alloy including this material, or zirconium oxide.

6. The functionalized implant according to claim 5, **characterized in that** a ceramic material, in particular an ATZ ceramic, a ZTA ceramic, or a zirconium oxide dominated ceramic with an yttrium oxide addition, or a metal is used as the substrate.

## Revendications

1. Procédé de fabrication d'un implant fonctionnalisé, en particulier d'un implant dentaire, comprenant les étapes suivantes :
a) fournir un substrat pour l'implant,
b) appliquer un revêtement très poreux et très hydrophile, au moins l'un des matériaux suivants étant tout d'abord appliqué pour la fonctionnalisation:
- du zirconium, du titane,
- un alliage de zirconium ou de titane, et
la couche de zirconium ou de titane ou l'alliage de zirconium ou de titane étant directement appliqué(e) sur la surface du substrat au moyen d'un procédé de dépôt physique en phase vapeur.

2. Procédé selon l'une des revendications précédentes, **caractérisé en ce que** le substrat revêtu est finalement placé dans une solution de NaOH, grâce à quoi la transformation chimique des couches en oxyde de titane ou en oxyde de zirconium est réalisée.

3. Procédé selon l'une des revendications précédentes, **caractérisé en ce qu'**une céramique est utilisée en tant que substrat, en particulier une céramique ATZ, une céramique ZTA ou une céramique dominée par de l'oxyde de zirconium avec un ajout d'oxyde d'yttrium.

4. Procédé selon la revendication 1 ou 2, **caractérisé en ce qu'**un métal est utilisé en tant que substrat.

5. Implant fonctionnalisé, en particulier implant dentaire, comprenant un substrat et un revêtement fonctionnalisé, adhérent et hydrophile qui présente au moins du zirconium, un alliage avec ce matériau ou de l'oxyde de zirconium.

6. Implant fonctionnalisé selon la revendication 5, **caractérisé en ce qu'**une céramique est utilisée en tant que substrat, en particulier une céramique ATZ, une céramique ZTA ou une céramique dominée par de l'oxyde de zirconium avec un ajout d'oxyde d'yttrium, ou un métal.
